# EUROPEAN PATENT APPLICATION

(11) **EP 1 484 073 A1**
(43) Date of publication of application: **08.12.2004**
(21) Application number: 03710338.9
(22) Date of filing: 13.03.2003
(51) Int. Cl.: A61M 5/36

(54) **RECOVERING IMPLEMENT**

(30) Priority: 14.03.2002 JP 2002070734
(71) Applicant: TERUMO KABUSHIKI KAISHA, Tokyo 151-0072 (JP)
(72) Inventor: TACHIKAWA, Kouichi, c/o Terumo kabushiki kaisha, Nakakoma-gun, Yamanashi 409-3853 (JP); KOYAMA, Shingo, c/o Terumo kabushiki kaisha, Nakakoma-gun, Yamanashi 409-3853 (JP); OKABE, Hiromitsu, c/o Terumo kabushiki kaisha, Tokyo 151-0072 (JP)
(74) Representative: Vollnhals, Aurel, Dipl.-Ing.
(86) International application number: PCT/JP2003/003010
(87) International publication number: WO 2003/075986

(57) **Abstract**

A sanitary recovering instrument capable of positively recovering, when excessive chemical liquid and interfusing gas are to be discarded from a liquid filled container pre-filled with a liquid and sealed, them into a closed space. The recovering instrument (1) in a preferable embodiment comprises a container body (2), a hollow needle tube (3), a gasket (4), an enclosing member (5), a cap (6), and a holder (7). When the needle tube (3) is allowed to puncture the elastic membrane (94) of the liquid-filled container (pre-filled syringe (8)), excessive chemical liquid and interfusing air are introduced into the storing space (20) in the container body (2) via the inner cavity (31) of the needle tube (3) to thereby move the gasket (4) slidingly and increase the volume of the space (20) according to the amount introduced.

## Description

### Technical Field

The present invention relates to a recovering instrument, and particularly to a recovering instrument for recovering excessive chemical liquid and interfusing gas from a liquid-filled container such as, for example, a pre-filled syringe.

### Background Art

Hitherto, as a form for containing a chemical or chemical liquid, there has been known a pre-filled syringe previously storing a chemical in a syringe.

The pre-filled syringe generally has a structure in which a syringe comprises an outer tube having a mouth portion wherein its diameter is reduced on the tip end side thereof, a gasket inserted in the outer tube via the base end opening of the outer tube, and a pusher (plunger rod) connected to the gasket, and a powdery chemical or chemical liquid is stored in a sealed condition in the space surrounded by the outer tube of the syringe and the tip end surface of the gasket of the syringe. The mouth portion is sealed with a sealing member such as an elastic membrane, and, when needed, the elastic membrane is punctured by a needle tube and the pusher is operated, whereby an infusion is introduced into the outer tube via the needle tube or a chemical liquid in the outer tube is discharged.

The pre-filled syringe is excellent in safety and simplicity, in view of that there is no need for an operation to transfer the chemical or chemical liquid stored therein into the syringe. Therefore, a shift to the pre-filled syringe, as a chemical storing form for replacing vial or ampule, has been contrived widely. In recent years, a shift to storage of a carcinostatic agent or the like in a pre-filled syringe has been desired and has come to be put to practical use.

Meanwhile, prior to the feeding of a chemical liquid in such a pre-filled syringe into an infusion bag or the dosage of the chemical liquid to the patient, a needle tube is attached to the pre-filled syringe, and air (bubbles) interfusing into the pre-filled syringe and excessive chemical liquid in the pre-filled syringe are discharged.

Conventionally, the chemical liquid and the like thus discharged are received by a tray or the like, and are not recovered into a closed space. However, where a chemical liquid not suited to jetting into the air, such as a carcinostatic agent, is contained in the pre-filled syringe, such a discharging operation is unsuitable for the operator.

### Disclosure of Invention

It is an object of the present invention to provide a recovering instrument capable of recovering, when excessive liquid or interfusing gas is to be discharged from a container pre-filled with a liquid, them into a closed space.

The above object can be attained by the present invention as characterized by the following paragraphs (1) to (10).
(1) A recovering instrument for recovering excessive liquid and interfusing gas from a liquid-filled container sealed with a sealing member and pre-filled with a liquid, including: a hard container body provided therein with a storing space for storing the excessive liquid and interfusing gas, and a needle tube connected on the base end side thereof to the container body, provided at the tip end thereof with a needle point capable of puncturing the sealing member, and having an inner cavity communicated with the storing space, wherein when the excessive liquid and interfusing gas are introduced into the storing space in the condition where the needle tube has punctured the sealing member and the inside of the liquid-filled container and the storing space are communicated with each other via the inner cavity of the needle tube, the volume of the storing space inside the container body is varied according to the amount introduced.
(2) A recovering instrument for recovering excessive liquid and interfusing gas from a liquid-filled container sealed with a sealing member and pre-filled with a liquid, including: a hard container body provided therein with a storing space for storing the excessive liquid and interfusing gas, a needle tube connected on the base end side thereof to the container body, provided at the tip end thereof with a needle point capable of puncturing the sealing member, and having an inner cavity communicated with the storing space, and a gasket slidably disposed inside the container body, wherein when the excessive liquid and interfusing gas are introduced into the storing space in the condition where the needle tube has punctured the sealing member and the inside of the liquid-filled container and the storing space are communicated with each other via the inner cavity of the needle tube, the gasket is moved slidingly, to thereby vary the volume of the storing space inside the container body, according to the amount introduced.
(3) A recovering instrument as set forth in the above paragraph (2), including a means for inhibiting the gasket from being released from the container body.
(4) A recovering instrument as set forth in the above paragraph (3), wherein the means is disposed at an end portion, on the opposite side of the needle tube, of the container body, and the means is either a cap provided with a vent hole through which the gasket cannot pass or a gas-permeable film.
(5) A recovering instrument as set forth in the above paragraph (3), wherein the means is a projection formed on the inside surface of an end portion, on the opposite side of the needle tube, of the container body.
(6) A recovering instrument as set forth in any of the above paragraphs (1) to (5), including a holder portion which is disposed so as to cover the periphery of the needle tube and which is capable of being mounted to or brought into contact with the liquid-filled container (particularly, capable of fitting or freely fitting of a liquid discharge portion of the liquid-filled container thereto).
(7) A recovering instrument as set forth in any of the above paragraphs (1) to (6), including an outflow preventive means for preventing the liquid recovered into the storing space from flowing out.
(8) A recovering instrument as set forth in the above paragraph (7), wherein the outflow preventive means is an enclosing member which is formed of an elastic material and which encloses the needle tube.
(9) A recovering instrument as set forth in any of the above paragraphs (1) to (8), including a contamination preventive means.
(10) A recovering instrument as set forth in any of the above paragraphs (1) to (9), wherein the liquid-filled container is a pre-filled syringe or a soft (flexible) container (e.g., bags).

### Brief Description of Drawings

FIG. 1 is a partly sectional plan view showing one embodiment (in a disassembled state) of the recovering instrument according to the present invention.
FIG. 2 is a partly sectional plan view showing the embodiment (in an assembled state) of the recovering instrument according to the present invention.
FIG. 3 is a vertical sectional view showing an operating condition in use of the recovering instrument shown in FIGS. 1 and 2.
FIG. 4 is a vertical sectional view showing an operating condition in use of the recovering instrument shown in FIGS. 1 and 2.

### Best Mode for Carrying Out the Invention

Now, the recovering instrument according to the present invention will be described in detail below, based on a preferred embodiment shown in the accompanying drawings.

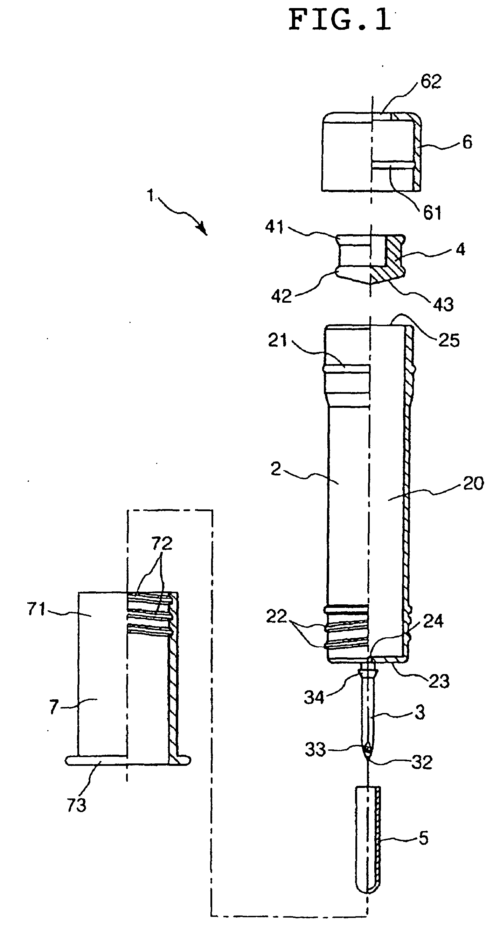

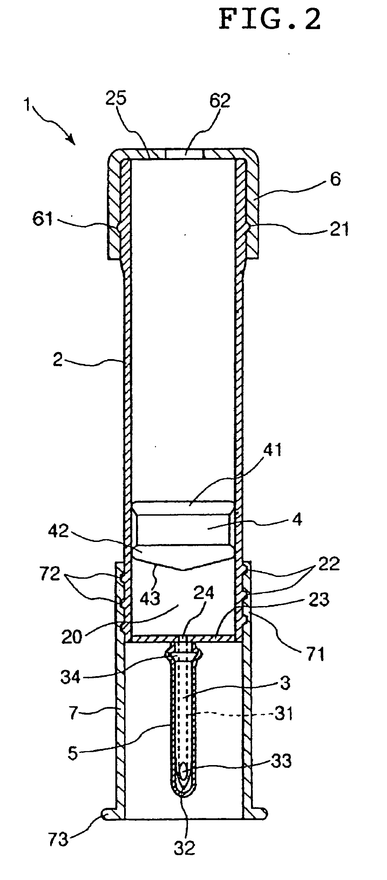

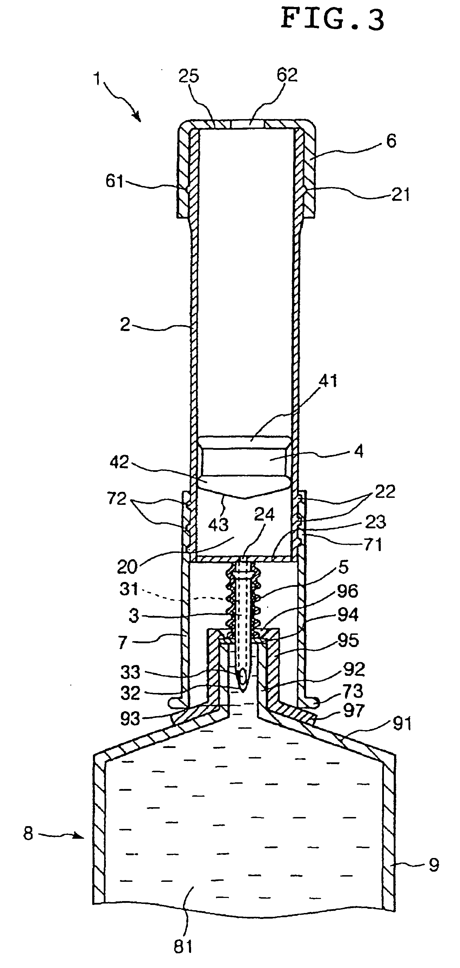

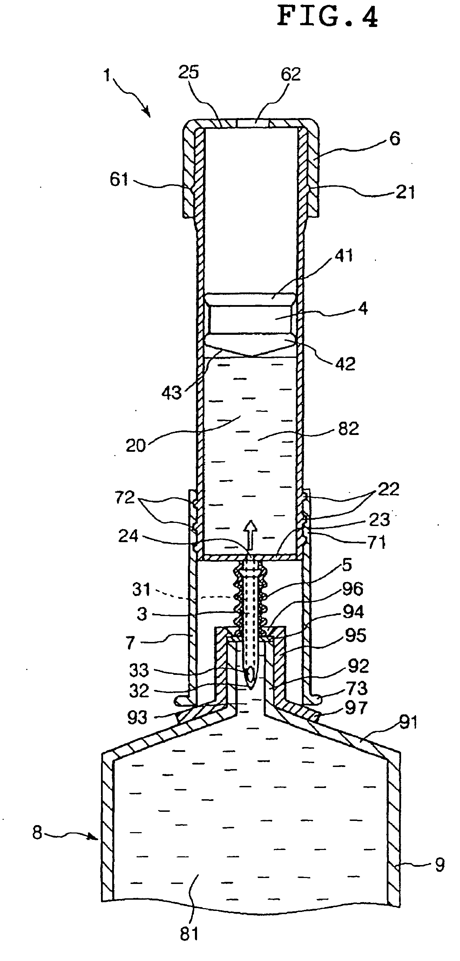

FIGS. 1 and 2 are partly sectional plan views showing one embodiment of the recovering instrument according to the present invention, in the disassembled state and in the assembled state, respectively, and FIGS. 3 and 4 are vertical sectional views showing an operation in use of the recovering instrument shown in FIGS. 1 and 2, respectively. Incidentally, for convenience of description, the upper side in FIGS. 1 to 4 will be referred to as "the base end", and the lower side as "the tip end".

The recovering instrument 1 according to the present invention is an instrument for recovering excessive liquid and interfusing gas (liquid and gas recovering instrument) from a liquid-filled container not including a needle tube in itself, for example, a syringe (pre-filled syringe) 8 shown in FIGS. 3 and 4. The recovering instrument 1 in this embodiment is composed of a hard container body 2, a needle tube 3 disposed at the tip end of the container body 2, a gasket 4 disposed slidably in the container body 2, an enclosing member 5 for enclosing the needle tube 3, a cap 6 mounted to a base end portion of the container body 2, and a holder 7 mounted to a tip end portion of the container body 2.

The container body 2 is in a bottomed tubular shape a base end portion 25 of which is open whereas the tip end side of which is closed with a bottom plate 23 having an opening 24 in its central portion. Examples of the material constituting the container body 2 include comparatively hard resin materials such as polyvinyl chloride, polyethylene, polypropylene, cyclic polyolefins, polystyrene, poly-(4-methylpentene-1), polycarbonate, acrylic resin, polysulfone, polyphenylsulfone, polyether sulfone, polyarylate resins, polymethyl methacrylate, acrylonitrile-butadiene-styrene copolymer, polyesters such as polyethylene terephthalate, and polyethylene naphthalate, butadiene-styrene copolymer, polyamides (e.g., nylon 6, nylon 6·6, nylon 6·10, nylon 12), etc., and various glass materials. Among the above materials, preferable are such resins as polycarbonate, polyesters, and cyclic polyolefins, in view of their easy moldability, high transparency, and high strength.

The container body 2 is provided, at the outer circumference of a base end portion thereof, with a ring-like projected portion 21 for fitting to the cap 6, over the entire circumference thereof.

In addition, the container body 2 is provided, at the outer circumference of a tip end portion thereof, with a male screw 22 for mounting the holder 7 thereto by screw engagement. Incidentally, a projected portion for fitting of the holder 7 may be provided on the circular circumference.

The needle tube (hollow needle) 3 is erected on a central portion of a bottom plate 23 of the container body 2, substantially perpendicular to the bottom plate 23. An inner cavity 31 of the needle tube 3 is communicated with a storing space 20 via the opening 24.

Incidentally, while the needle tube 3 is formed of the same resin material as that for the container body 2 integrally with the container body 2 in this embodiment, the needle tube 3 is not limited to such one but may be a needle tube which is formed of a metal, for example, stainless steel, and which is attached to the container body 2.

A needle point 32 of the needle tube 3 is provided with a side hole 33. The side hole 33 is communicated with the inner cavity 31 of the needle tube 3.

The needle tube 3 is provided, on the outer circumference of the base end portion thereof, with a shade-like shaped barb 34 for preventing the enclosing member 5 from slipping off from the needle tube 3.

The gasket 4 formed of an elastic material is stored in the container body 2. The tip end surface 43 of the gasket 4 and the bottom plate 23 define the storing space 20, for a liquid and the like, in the container body 2.

The gasket 4 is moved slidingly in the container body 2 along the longitudinal direction of the container body 2 by the liquid pressure (or gas pressure) of a liquid (and gas) 82 introduced from the syringe 8 via the inner cavity 31 of the needle tube 3. Therefore, the volume of the storing space 20 surrounded by the container body 2 and the tip end surface 43 of the gasket 4 is varied according to the amount of the liquid (and gas) introduced.

The gasket 4 is provided at an outer circumferential portion thereof with a plurality of ring-like projected portions 41, 42 along the entire circumference. The projected portions 41, 42 are moved slidingly while keeping close contact with the inner circumferential surface of the container body 2, whereby the liquid-tightness of the storing space 20 is securely maintained, and the sliding performance of the gasket 4 can be enhanced.

In this embodiment, two projected portions 41, 42 are formed along the longitudinal direction of the gasket 4. Specifically, the projected portions 41, 42 are formed respectively at the base end portion and tip end portion of the gasket 4. Incidentally, the locations, the number, the sectional shape and the like of the projected portions 41, 42 are not limited to those shown in the figures.

The material constituting the gasket 4 is not particularly limited, as long as the material permits the gasket 4 to be moved slidingly in the container body 2. Examples of the material include elastic materials such as various rubber materials such as natural rubber, butyl rubber, isoprene rubber, butadiene rubber, styrene-butadiene rubber, silicone rubbers, etc., various thermoplastic elastomers such as polyurethane-based, polyester-based, polyamide-based, olefin-based, and styrene-based ones, mixtures of the above-mentioned materials, etc. In addition, laminated materials produced by laminating a film, such as a fluorocarbon resin film, on these elastic materials may also be used as the material for constituting the gasket 4.

Incidentally, it suffices that at least an outer circumferential portion of the gasket 4 is formed of one of the above-mentioned elastic materials. For example, a structure may be adopted in which the gasket 4 has a core portion (not shown) constituted of a resin material, and the elastic material is so arranged as to cover the outer circumference of the core portion.

The enclosing member 5 for enclosing the needle tube 3 is disposed in the surroundings of the needle tube 3. The enclosing member 5 has the function of preventing pollution of the needle tube 3 in no-use time (storage time) (the prevention of pollution of the needle tube 3 leads to the prevention of pollution of a chemical liquid 81) and the function of preventing the excessive liquid and the like recovered into the storing space 20 from leaking out through the side hole 33 of the needle point 32 to be scattered into the exterior. In this meaning, the enclosing member 5 is a pollution preventing means for preventing pollution of the needle tube 3 and is also an outflow preventive means for preventing the liquid and the like from flowing out.

In this embodiment, the enclosing member 5 functions as both the pollution preventive mans and the outflow preventive means. This results in that the prevention of pollution and the prevention of the outflow of the liquid can be effectively achieved with a small-sized lightweight simple structure (a reduced number of component parts).

As the enclosing member 5, preferably used are enclosing members which are composed of a membrane formed of any of various rubber materials (elastic materials) such as natural rubber, butyl rubber, isoprene rubber, butadiene rubber, styrene-butadiene rubber, silicone rubbers, latex rubber, and mixtures thereof, and which can be easily punctured by the needle point 32.

The base end portion of the enclosing member 5 extends beyond the barb 34 formed on the outer circumference of the needle tube 3 to the vicinity of the bottom plate 23. In this case, the base end portion of the enclosing member 5 may be simply fitted to (in close contact with) the needle tube 3, but it is preferable that the base end portion is attached to the needle tube 3 by adhesion with an adhesive, fusing or the like method.

Incidentally, another example of the pollution preventive means may be, for example, of a configuration in which the needle tube 3 is covered with a film, and the film is unsealed at the time of use of the needle tube 3. In addition, another example of the outflow preventive means may be, for example, of a configuration in which a check valve (one-way valve) is disposed in the vicinity of the opening 24.

The cup-shaped cap 6 is fitted over the base end portion of the container body 2. The cap 6 is a means (member) for inhibiting the gasket 4 from being released from the base end of the container body 2.

The cap 6 is provided at its inner circumferential surface with a ring-like recessed portion 61 for fitting on the projected portion 21. The cap 6 is mounted to the container body 2 in the condition where the projected portion 21 and the recessed portion 61 are fitted to each other. In this case, the cap 6 may be simply fitted to the container body 2; however, for secure prevention of the release, it is preferable that the container body 2 and the cap 6 are attached to each other by adhesion with an adhesive, fusing or the like method, after the insertion of the gasket 4 in the container body 2.

In addition, the cap 6 is provided, in a central portion of the base end thereof, with a roughly circular opening (vent hole) 62, to enable ventilation. The inside diameter of the opening 62 is smaller than the outside diameter of the gasket 4, so that the gasket 4 cannot pass through the opening 62. Incidentally, the shape, formation position, number and the like of the opening(s) 62 are naturally not limited to those shown in the figures.

For example, in another form in which the cap 6 is not used, a gas-permeable film may be fused to the base end portion 25 of the container body 2. Besides, a projection or projections may be formed on the inside surface of the base end portion 25.

The holder (holder portion) 7 is detachably attached to the tip end portion of the container body 2. The holder 7 can be mounted to or brought into contact with the syringe 8 which will be described later.

The holder 7 is constituted of a tubular member opened at both ends thereof, and is provided at its base end portion with a connection portion 71 for connection to the container body 2. The inner circumferential surface of the connection portion 71 is provided with a female screw 72 for screw engagement with the above-mentioned male screw 22. Incidentally, both of the members may be fitted to each other; in that case, the recessed portion may be provided on the circular circumference.

In addition, a flange 73 is formed on the outer circumference of the tip end of the holder 7. The flange 73 abuts on (or approaches) the syringe 8 or an adjunct thereof at the time of connection with the syringe 8 which will be described later.

In the condition where the holder 7 has been mounted to the container body 2, namely, in the condition where the male screw 22 and the female screw 72 are in screw engagement with each other (see FIG. 2), the holder 7 is so disposed as to cover (surround) the surroundings of the needle tube 3 and the enclosing member 5 in a non-contact manner. In this case, the tip end of the enclosing member 5 (the tip end of the needle tube 3) is not protruding to the tip end side beyond the tip end of the holder 7, and is located on the inside of the holder 7. This ensures that the needle point 32 and the tip end portion of the enclosing member 5 are protected.

Incidentally, in the present invention, the holder portion such as the holder 7 may be attached to the container body 2 or may be integrated with the container body 2. In addition, the shape of the holder portion is not limited to the tubular shape shown in the figures; for example, the shape may be a frame-like shape.

The recovering instrument 1 as above-described is preferably subjected to a sterilizing treatment over the inside thereof or the entire part thereof.

Next, the use method (functions) of the recovering instrument 1 will be described referring to FIGS. 3 and 4.

The recovering instrument 1 is used for recovering an excessive portion of a chemical liquid in a liquid-filled container not including a needle tube in itself, such as, for example, a syringe (pre-filled syringe) 8 shown in FIG. 3.

The syringe 8 is a pre-filled syringe with the chemical liquid 81 previously stored therein, and includes an outer tube (syringe outer tube) 9, a gasket (not shown) capable of being moved slidingly in the outer tube 9, and a pusher (not shown) which is connected to the gasket and which operates the gasket to move.

A reduced diameter portion 92 reduced in diameter relative to the trunk portion of the outer tube 9 is formed integrally with a roughly central portion of a bottom portion (shoulder portion) 91 of the outer tube 9. A membrane (sealing member) 94 formed of an elastic material is mounted to the base end (the top end in FIG. 3) of the reduced diameter portion 92, to seal gas-tight the inside cavity 93 of the reduced diameter portion 92. The membrane 94 can be punctured by the needle tube 3. It is preferred that the membrane 94 has the self-closability of the punctured hole. In the case of the membrane 94 having the self-closability of the punctured hole, when the puncturing needle tube 3 is drawn out of the membrane 94, the punctured hole is closed due to the restoring property of the membrane 94, whereby the chemical liquid 81 is inhibited from leaking out.

In addition, a stationary member 95 is fitted over and fixed to the outside of the reduced diameter portion 92. The stationary member 95 is provided with an opening 96 in its base end, and an outer circumferential portion of the membrane 94 is clamped between an edge portion of the opening 96 and the tip end surface of the reduced diameter portion 92, whereby the membrane 94 is fixed in a liquid-tight manner.

The stationary member 95 can be inserted (fitted or freely fitted) in the holder 7, and the outside diameter of a principal portion (body portion) of the stationary member 95 is not more than the inside diameter of the holder 7.

Besides, the stationary member 95 is provided, on the outer circumference of a tip end portion thereof, with a spread portion (flange) 97 spread in a skirt shape. The spread portion 97 is joined to the bottom portion 91 of the outer tube 97.

Incidentally, the membrane 94 may be replaced by a plug body formed of an elastic material.

Specific examples of the chemical liquid 81 include vitamin preparations (general vitamin preparations), various amino acids, antithrombotic such as heparin, insulin, antibiotic, carcinostatic agents, antitumor agents, analgesic, cardiac, intravenous anesthetic, antiparkinsonism agents, antiulcer agents, cortical hormone agents, antiarrhythmic, correcting electrolytes, etc. In addition, a chemical in a solid state (e.g., powdery form, tablet form) may be preserved in the syringe 8 and may be dissolved in a liquid such as infusion to form the chemical liquid before use. Incidentally, in the present invention, the chemical liquid 81 is naturally not limited to the above-mentioned. In addition, the chemical liquid 81 may be a liquid without drug efficacy, for example, distilled water.

In the case of recovering an excessive portion of the chemical liquid 81 in the syringe 8 as above-mentioned, first, the holder 7 is mounted to the syringe 8 in the condition where the holder 7 is in screw engagement with the container body 2 of the recovering instrument 1. The mounting is conducted by inserting the stationary member 95 of the syringe 8 into the holder 7 via the tip end opening of the holder 7.

As the stationary member 95 and the reduced diameter portion 92 of the syringe 8 are inserted into the holder 7, the tip end of the enclosing member 5 comes into the opening 96, and makes contact with the membrane 94. A further insertion permits the needle point 32 of the needle tube 3 to puncture the membrane 94. Prior to the puncturing, the needle point 32 punctures the enclosing member 5, and, thereafter, the enclosing member 5 is pushed in the direction of the base end by receiving a reaction force from the membrane 94, is put into a bellows-like compressed state, and is maintained in the compressed state (see FIG. 3).

When the flange 73 of the holder 7 is brought into contact with the spread portion 97 of the stationary member 95, the mounting of the holder 7 to the syringe 8 is completed. In this condition, the depth of insertion of the needle tube 3 into the reduced diameter portion 92 is in such an extent that at least the side hole 33 of the needle tube 3 comes beyond the membrane 94, to be located in the inner cavity 93 of the reduced diameter portion 92.

Here, where the length of the needle tube 3 is constant, the depth of insertion of the needle tube 3 into the syringe 8 is determined by the length of the holder 7 (precisely, the length of the portion of the holder 7 exclusive of the connection portion 71 for connection to the container body 2). Therefore, by appropriately selecting the holders 7 different in length and mounting the selected holders 7, the holders 7 can be used in optimum conditions for various syringes, particularly various syringes different in the length of the reduced diameter portion 92.

In addition, the needle tube 3, after puncturing the membrane 94, enters into the inner cavity 93 of the reduced diameter portion 92, to make contact with the chemical liquid 81. Since the needle tube 3 is covered with the enclosing member 5 until the time point immediately before the puncturing through the membrane 94 and pollution of the needle tube 5 is prevented, there is no possibility that the polluted needle tube makes contact with the chemical liquid 81 so as to produce bad effects.

In the condition where the base end of the recovering instrument 1 is directed vertically upwards, the pusher (not shown) of the syringe 8 is pushed with a finger, and the gasket (not shown) in the outer tube 9 is moved in the direction of the base end. As a result, the chemical liquid 81 in the syringe 8 passes through the side hole 33 and the inner cavity 31 of the needle tube 3, to be discharged into the storing space 20. Incidentally, where air (bubbles) is left in the syringe 8, the air is discharged and fed into the storing space 20 together with the chemical liquid 81.

Attendant on the discharge of the chemical liquid 81 into the storing space 20, namely, attendant on the introduction of the excessive chemical liquid 82 into the storing space 20, the pressure inside the storing space 20 is increased, and the gasket 4 is pushed and moved (slid) in the direction of the base end. This causes an increase in the volume of the storing space 20 (see FIG. 4).

When a desired amount of excessive chemical liquid 82 (and air) has been recovered into the storing space 20, the operation of pushing the pusher of the syringe 8 is stopped, and the recovering instrument 1 is detached from the syringe 8. This causes the needle tube 3 to be drawn out of the membrane 94.

When the needle tube 3 is drawn out of the membrane 94, the bellows-like compressed enclosing member 5 instantaneously restores its original shape, and again encloses the needle point 32. This prevents the excessive chemical liquid 82 recovered into the storing space 20 from leaking out through the side hole 33 to be scattered to the surroundings.

In addition, since the hole punctured by the needle tube 3 is automatically closed due to the restoring property of the membrane 94 (self-closability of the punctured hole) as has been described above, leaking-out of the chemical liquid 81 is prevented also on the side of the syringe 8.

When the operation of recovering the liquid and the like into the recovering instrument 1 is finished in the above-mentioned manner, the excessive chemical liquid 82 recovered is discarded together with the recovering instrument 1. Thus, the excessive chemical liquid 82 can be discarded sanitarily, without being scattered to the surroundings or making contact with the worker.

Incidentally, at least a part of the excessive chemical liquid 82 recovered into the recovering instrument 1 may be served to reutilization, as required.

In the present invention, the liquid-filled container is not limited to the syringe previously filled with a liquid (pre-filled syringe), and other forms may be adopted, for example, soft containers (infusion bags, etc.).

Besides, the liquid to be recovered is not limited to the chemical liquid, and may be any of other liquids such as distilled water, physiological saline, cleaning liquid, etc.

While the recovering instrument according to the present invention has been described above referring to the embodiment shown in the figures, the present invention is not limited to the embodiment; the configuration of each component or part may be replaced by an arbitrary configuration capable of displaying the same function as above-mentioned, and arbitrary configurations may be added.

### Industrial Applicability

As has been described above, according to the recovering instrument of the present invention, a liquid and a gas can be recovered into a closed space and then discarded, which does not cause pollution of the surroundings and which is effective particularly for discarding liquids unsuitable for ejection into the air, such as carcinostatic agents.

In addition, where a configuration is adopted in which the volume of the container is varied by the movement of the gasket, the introduction resistance of the liquid and the like is constant, so that the recovering operation can be conducted easily and securely, without causing a reverse movement of the liquid and the like recovered, and it is easy to check the amount of the liquid and the like recovered.

Besides, with the holder portion provided, recovery of a liquid and the like can be performed in more suitable conditions, for example, the depth of insertion of the needle tube into the liquid-filled container (syringe or the like) can be determined, and this ensures that the tip end portions of the needle point 32 and the enclosing member 5 are protected.

In addition, where the outflow preventive means is provided, the recovered liquid can be more securely prevented from leaking out.

Besides, where the pollution preventive means is provided, pollution of the chemical liquid can be prevented effectively.

## Claims

1. A recovering instrument for recovering excessive liquid and interfusing gas from a liquid-filled container sealed with a sealing member and pre-filled with a liquid, comprising:
a hard container body provided therein with a storing space for storing said excessive liquid and interfusing gas, and
a needle tube connected on the base end side thereof to said container body, provided at the tip end thereof with a needle point capable of puncturing said sealing member, and having an inner cavity communicated with said storing space, wherein
when said excessive liquid and interfusing gas are introduced into said storing space in the condition where said needle tube has punctured said sealing member and the inside of said liquid-filled container and said storing space are communicated with each other via said inner cavity of said needle tube, the volume of said storing space 20 inside said container body is varied according to the amount introduced.

2. A recovering instrument for recovering excessive liquid and interfusing gas from a liquid-filled container sealed with a sealing member and pre-filled with a liquid, comprising:
a hard container body provided therein with a storing space for storing said excessive liquid and interfusing gas, a needle tube connected on the base end side thereof to said container body, provided at the tip end thereof with a needle point capable of puncturing said sealing member, and having an inner cavity communicated with said storing space, and a gasket slidably disposed inside said container body, wherein
when said excessive liquid and interfusing gas are introduced into said storing space in the condition where said needle tube has punctured said sealing member and the inside of said liquid-filled container and said storing space are communicated with each other via said inner cavity of said needle tube, said gasket is moved slidingly, to thereby vary the volume of said storing space 20 inside said container body, according to the amount introduced.

3. A recovering instrument as set forth in claim 2, comprising means for inhibiting said gasket from being released from said container body.

4. A recovering instrument as set forth in any of claims 1 to 3, comprising a holder portion disposed so as to cover the periphery of said needle tube and capable of being mounted to said liquid-filled container.

5. A recovering instrument as set forth in any of claims 1 to 4, comprising outflow preventive means for preventing said liquid recovered into said storing space from flowing out.

6. A recovering instrument as set forth in claim 5, wherein said outflow preventive means is an enclosing member which is formed of an elastic material and which encloses said needle tube.
